# EUROPEAN PATENT APPLICATION

(11) **EP 4 821 923 A1**
(43) Date of publication of application: **14.10.2026**
(21) Application number: 25168785.1
(22) Date of filing: 07.04.2025
(51) Int. Cl.: C07D 307/68

(54) **METHOD FOR PREPARING 4-AMINO-FURANE**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present invention relates to a novel method for preparing 4-amino-furanes of the general formula (I) and salts thereof.

## Description

The present invention relates to a novel method for preparing 4-amino-furanes of the general formula (I) and salts thereof.

4-Amino-furanes of the general formula (I) (especially R¹= COOMe) are important precursors of agrochemical active ingredients (cf. WO2018/228985) and pharmaceutical active ingredients (e.g. DNA binding agents: Woods, Craig R. et al. Bioorganic & Medicinal Chemistry Letters, 12(18), 2647-2650; 2002).

4-Amino-furanes of the general formula (I) serve as starting material for the preparation of tetrahydro- and dihydrofurane carboxylic acids and esters. To date, these compounds of the formula (I) have been prepared by a multi-stage synthesis including a bromination, dehalogenation, coupling reaction and deprotection. (see F. Brucoli, et al. Bioorganic & Medicinal Chemistry, 20(6), 2019-2024; 2012).

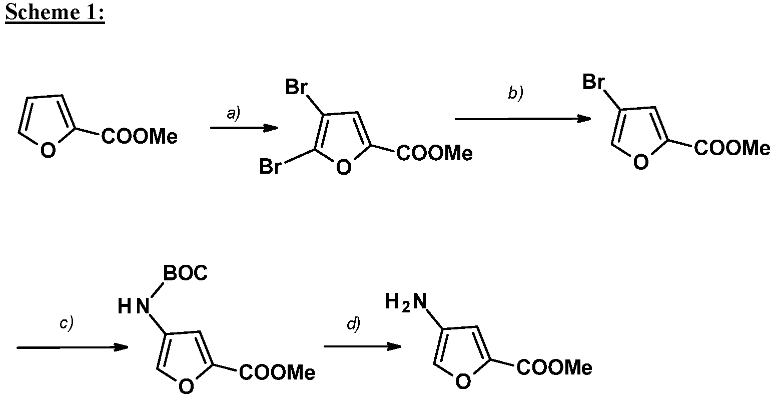

Br₂, AlCl₃; b) Zn, NH₄Cl; c) Cul/ (CH₃NHCH₂)₂, Boc-NH₂, K₂CO₃ d) removal of Boc protecting group

The synthesis mentioned above has a large number of disadvantages, such as low atom economy (bromination and dehalogenation), use of heavy metals such as zinc and use of protecting groups such as Boc-amine. The method described in Bioorganic & Medicinal Chemistry, 20(6), 2019-2024; 2012 furthermore requires the use of metal-containing (for example copper(I) iodide) catalysts.

These disadvantages render the method for preparing compounds of the general formula (I) uneconomic and therefore very expensive.

F. Wolter et al in (Organic Letters, 11(13), 2804-2807; 2009) describes another method for preparing aminofurans of the general formula (I), specifically via a Curtius rearrangement of dimethyl furan-2,4-dicarboxylate using (PhO₃)₂P(O)N₃. This method is unsuitable for industrial applications due to the highly explosive properties of organic azides.

Several compounds of the general formula (I), for example where R¹= CF₃ and R²= NHAryl, have been described in European Journal of Organic Chemistry 2018, 3853-3861. However, this compound was detected in a mixture of several components.

WO 2022/018057 describes a synthesis in the presence of a dehydrating reagent which can be used on industrial scale, e.g. thionyl chloride, oxalyl chloride or phosphorous oxychloride. However, these dehydrating reagents have different disadvantages for the process and in addition for product of the general formula (I).

The disadvantage of thionyl chloride is the presence of sulphur in the product - the compounds of the general formula (I) - which poisons catalysts in further reactions. As the compound of formula (I) is an important precursor of agrochemical and pharmaceutical active ingredients it is often further reacted, and this disadvantage is of major importance.

The disadvantage of oxalyl chloride is the moderate yield, higher costs of the reagent and potential formation of low solubility by-products which would lead to significantly increased purification requirements.

The use of phosphorous oxychloride would lead to the formation of stoichiometric phosphorous waste which is not environmentally friendly and cause high purification efforts and disposal costs.

In light of the prior art, the object of the present invention is to find a sulphur-free, cost-effective and environmentally friendly method for preparing compound of formula (**I**) and which is therefore more favourable to be used in industry. It is also desirable to obtain these compounds with high yield and at high purity, such that they do not have to be subjected to any further complex purification.

The object described above - simple, cost-effective, sulphur-free, environmentally friendly and large-scale production - is achieved by a method for preparing compounds of the general formula (I) and salts thereof 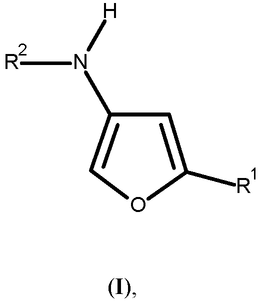 in which
R¹ is CF₃, CF₂H, C₂F₅, CF₂Cl, CCl₃, COOR³, COOH,
R² is H, CH₃CO, CCl₃CO, CF₃CO, phenyl-CO, CH₃OCO, (CH₃)₃COCO, phenyl, phenyl-CH₂, (diphenyl)CH,
R³ is (C₁-C₄)alkyl,
characterized in that
compounds of the general formula (II) 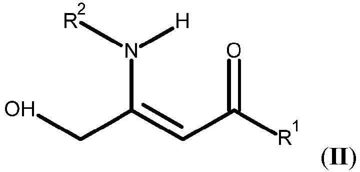
with R¹ and R² are as defined above
are reacted in the presence of phosgene or triphosgene to give compounds of the general formula (I).

Preferred definitions of the radicals of the compounds of the general formulae (I), (II) are as follows:
- R¹: is CF₃, CF₂H, CF₂Cl, C₂F₅, CCl₃, COOR³,
- R²: is H, CF₃CO, CH₃CO, CCl₃CO, phenyl, phenyl-CH₂, (diphenyl)CH, CH₃OCO, (CH₃)₃COCO,
- R³: is Methyl, Ethyl.

Particularly preferred definitions of the radicals of the compounds of the general formulae (I), (II) are as follows:
- R¹: is CF₃, CF₂H, CCl₃, COOR³,
- R²: is H, COCF₃, COCH₃, COCCl₃, Ph-CH₂, (diphenyl)CH, CH₃OCO, (CH₃)₃COCO,
- R³: is Methyl, Ethyl.

Especially preferred definitions of the radicals of the compounds of the general formulae (I), (II) are as follows:
- R¹: is CF₃, COOR³,
- R²: is H, COCF₃, (CH₃)₃COCO,
- R³: is Methyl, Ethyl.

Further especially preferred definitions of the radicals of the compounds of the general formulae (I), (II) are as follows:
- R¹: is COOR³,
- R²: is H,
- R³: is Methyl, Ethyl.

Further especially preferred definitions of the radicals of the compounds of the general formulae (I), (II) are as follows:
- R¹: is COOR³,
- R²: is H.
- R³: is Methyl.

The reaction sequence for preparing compounds of the formula (I) is shown in Scheme 2:

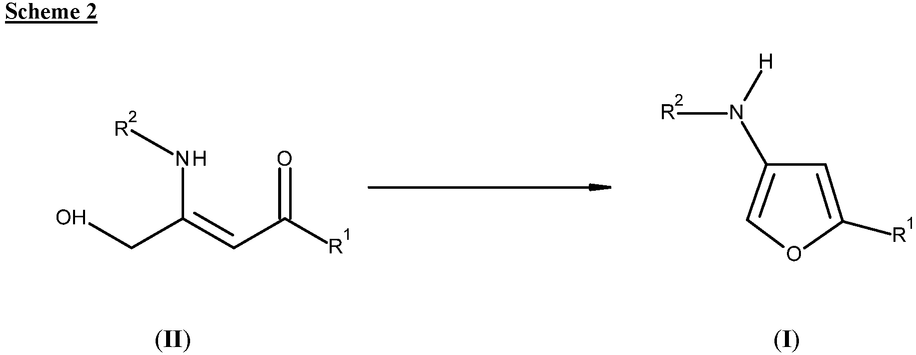

Compounds of the formula (II) react with phosgene or triphosgene to form compounds of the general formula (I). The preparation of compounds of the formula (II) are known from WO 2022/018057.

Compounds of the formula (II) are cyclized. The ring closure takes place in the presence of a dehydrating reagent phosgene or triphosgene.

Compounds of the formula (I) are formed in the form of HCl salts thereof.

If the compounds of the general formula (I) are obtained in the form of salts thereof, for example as a hydrochloride, the salt-free forms can be obtained by treating the salt with a base, for example triethylamine (see also WO 2022/018057).

The molar ratio of the compound of the formula (II) to the cyclization reagent is in the range of about 1:0.1 to 1:5, preferably from 1:0.5 to 1:3.

The reaction is usually carried out in a temperature range of -10°C to 40°C and optionally in the presence of a solvent or diluent. The reaction is preferably carried out in a solvent at -5 °C to 20 °C.

Preferred solvents are toluene, xylenes, chlorobenzene, R³OCOOR³ or R³OH (if R¹ = COOR³ in which R³ is (C₁-C₄)alkyl), or mixtures thereof.

If R¹ is an ester, the respective solvent is determined by the type of ester in the final product, e.g: dimethyl carbonate or methanol as solvent finally refers to COOMe.

Especially preferred solvents are toluene, xylenes, dimethyl carbonate and methanol or mixtures thereof.

### Description of the method

### Examples

The present invention is elucidated in more detail by the examples which follow, without restricting the invention to these examples.

### Method of measurement

The products were characterized by ¹H NMR spectroscopy and/or HPLC.

The NMR spectra were determined using a Bruker Avance II 600.

The product assay determination by quantitative HPLC were measured on an Agilent Technologies HP 1100 using Zorbax Eclipse 5 XDB-C8 column (150*4.6 mm) and acetonitrile, aqueous buffer gradient. Methyl 4-aminofuran-2-carboxylate hydrochloride (prepared, purified as analytical sample) was used as references substance.

### Example 1

### Methyl 4-aminofuran-2-carboxylate hydrochloride (salt of formula (I)) using triphosgene

20 g (95.4 wt.%, 120 mmol, 1.0 eq) of methyl 4-amino-5-hydroxy-2-oxo-pent-3-enoate and 50 ml (39.6 g) of methanol were added to a jacketed reactor and the suspension was stirred at 0 °C. A solution of 25.7 g (86.8 mmol, 0.72 eq) triphosgene, dissolved in 80 ml (69.2 g) of toluene, was dosed over 1 h and the temperature was maintained at 0-5°C. After complete dosing, stirring continued for 19 h at 0 °C. The suspension was filtered, the filter cake washed with 30 ml of a solvent mixture of 11,4 ml (9 g) methanol and 18,6 ml (16,1 g) toluene and dried under reduced pressure. 19.6 g (98.1 wt.%, 90% yield) of the product was obtained as a beige solid.

¹H-NMR (600 MHz, d6-DMSO): δ 9.00-11.00 (3H, br.); 8.08 (1H, d); 7.31 (1H, d); 3.83 (3H, s) ppm.

¹³C-NMR (600 MHz, d6-DMSO): 158.0; 143.6; 140.3; 121.7; 114.8; 112.1; 52.4 ppm.

### Example 2

### Methyl 4-aminofuran-2-carboxylate hydrochloride (salt of formula (I)) using phosgene

20 g (95.4 wt.%, 120 mmol, 1.0 eq) of methyl 4-amino-5-hydroxy-2-oxo-pent-3-enoate, 50 ml (39.6 g) of methanol and 80 ml (69.2 g) of toluene were added to a jacketed reactor and the suspension was stirred at 0 °C. 30,84 g (311 mmol) phosgene gas were dosed into the suspension through a gas inlet tube over 1 h and the temperature was maintained at 0-5°C. After complete dosing, stirring continued for 19 h at 0 °C. The suspension was filtered, the filter cake washed two times with 30 ml of a solvent mixture of 11,4 ml (9 g) methanol and 18,6 ml (16,1 g) toluene and dried under reduced pressure. 19.9 g (97,4 wt.%, 91,4% yield) of the product was obtained as a beige solid.

¹H-NMR (600 MHz, d6-DMSO): δ 9.00-11.00 (3H, br.); 8.12 (1H, d); 7.33 (1H, d); 3.83 (3H, s) ppm.

### Example 3

### Methyl 4-aminofuran-2-carboxylate hydrochloride (salt of formula (I)) using triphosgene

In an argon flushed reactor 20 g (94.7 wt.%, 119 mmol, 1.0 eq) of methyl 4-amino-5-hydroxy-2-oxo-pent-3-enoate were suspended in 39.6 g of Methanol and cooled to 0 °C to 5 °C. To this suspension a solution of 25.7 g (86.8 mmol, 0.72 eq) triphosgene, which was dissolved in 64.1 g of dimethyl carbonate, was added over 1 h and the temperature was maintained at 0-5°C. After complete dosing, stirring was continued for 20 h at 0 °C to 5 °C. Then, the suspension was filtered, the filter cake was washed with dimethyl carbonate and the obtained solid was dried under reduced pressure. 19.7 g (97.6 wt.%, 91% yield) of the product were obtained as a brownish solid.

### Example 4

### Methyl 4-aminofuran-2-carboxylate hydrochloride (salt of formula (I)) using triphosgene

In an argon flushed reactor 20 g (94.7 wt.%, 119 mmol, 1.0 eq) of methyl 4-amino-5-hydroxy-2-oxo-pent-3-enoate were suspended in a mixture of 31.7 g Methanol and 7.9 g dimethyl carbonate and cooled to 0 °C to 5 °C. To this suspension a solution of 25.7 g (86.8 mmol, 0.72 eq) triphosgene, which was dissolved in 64.1 g of dimethyl carbonate, was added over 1 h and the temperature was maintained at 0-5°C. After complete dosing, stirring was continued for 20 h at 0 °C. to 5 °C. Then, the suspension was filtered, the filter cake was washed with dimethyl carbonate and the obtained solid was dried under reduced pressure. 19.9 g (97.58 wt.%, 92% yield) of the product were obtained as a brownish solid.

### Example 5

### Comparative example:

### Methyl 4-aminofuran-2-carboxylate hydrochloride (salt of formula (I)) using thionyl chloride

50.0 g (97 wt.%, 0.3 mol, 1.0 eq) of methyl 4-amino-5-hydroxy-2-oxo-pent-3-enoate were placed in 113 g of methanol and the suspension was cooled to 0 °C. At 0-5 °C, 34.1 g (0.28 mol, 0.94 eq) thionyl chloride were dosed over 2 h. After complete dosing, stirring continued for 16 h at 0 °C and the suspension was then cooled to -7 °C. The solid was filtered, the filter cake was washed twice at -5 °C with 37 g methanol each and dried. 49.7 g (>99 wt.% (by qHPLC), 92% yield) of the product was obtained as a beige solid. The sulphur content was determined to be 160 ppm by elemental analysis.

### Example 6

### Comparative example:

### Methyl 4-aminofuran-2-carboxylate hydrochloride (salt of formula (I)) using oxalyl chloride

82.3 g (94 wt.%, 0.5 mol, 1.0 eq) of methyl 4-amino-5-hydroxy-2-oxo-pent-3-enoate were placed in 330 g of methanol and the suspension was cooled to 0 °C. At 0-5 °C, 86.1 g (0.52 mol, 1.05 eq) oxalyl chloride, dissolved in 86.1 g toluene, was dosed over 3 h. After complete dosing, stirring continued for 16 h at 0 °C, the suspension was then heated to 20°C and stirred at this temperature for 30 min. Finally, the suspension was tempered to -5 °C for 2 h, filtered, the filter cake washed twice at -5 °C with 60 g methanol each and dried. 69.2 g (93.3 wt.% (by qHPLC), 73% yield) of the product was obtained as a beige solid.

## Claims

1. Method for preparing compounds of the general formula (I) and salts thereof, 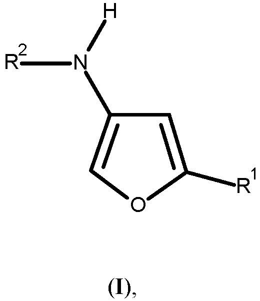 in which
R¹ is CF₃, CF₂H, C₂F₅, CF₂Cl, CCl₃, COOR³, COOH,
R² is H, CH₃CO, CCl₃CO, CF₃CO, phenyl-CO, CH₃OCO, (CH₃)₃COCO, phenyl, phenyl-CH₂, (diphenyl)CH,
R³ is (C₁-C₄)alkyl,
**characterized in that** compounds of the general formula (II) 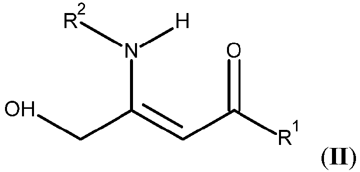 with R¹ and R² are as defined above are reacted in the presence of phosgene or triphosgene to give compounds of the general formula (I).

2. Method according to Claim 1, **characterized in that** the definitions of the radicals of the compounds of the general formulae (I) and (II) are as follows:
R¹ is CF₃, CF₂H, CF₂Cl, C₂F₅, CCl₃, COOR³,
R² is H, CF₃CO, CH₃CO, CCl₃CO, phenyl, phenyl-CH₂, (diphenyl)CH, CH₃OCO, (CH₃)₃COCO,
R³ is Methyl, Ethyl.

3. Method according to Claim 1, **characterized in that** the definitions of the radicals of the compounds of the general formulae (I) and (II) are as follows:
R¹ is CF₃, CF₂H, CCl₃, COOR³,
R² is H, COCF₃, COCH₃, COCCl₃, Ph-CH₂, (diphenyl)CH, CH₃OCO, (CH₃)₃COCO,
R³ is Methyl, Ethyl.

4. Method according to Claim 1, **characterized in that** the definitions of the radicals of the compounds of the general formulae (I) and (II) are as follows:
R¹ is CF₃, COOR³,
R² is H, COCF₃, (CH₃)₃COCO,
R³ is Methyl, Ethyl.

5. Method according to Claim 1, **characterized in that** the definitions of the radicals of the compounds of the general formulae (I) and (II) are as follows:
R¹ is COOR³
R² is H,
R³ is Methyl, Ethyl.

6. Method according to Claim 1, **characterized in that** the definitions of the radicals of the compounds of the general formulae (I) and (II) are as follows:
R¹ is COOR³,
R² is H.
R³ is Methyl.

7. Method according to any of Claims 1 to 6, **characterized in that** the reaction is carried out at -5 °C to 20 °C.

8. Method according to any of Claims 1 to 7, **characterized in that** the solvent is toluene, xylenes, dimethyl carbonate and methanol or mixtures thereof.
